# FASCICULE DE BREVET EUROPEEN

(11) **EP 0 771 171 B1**
(45) Date de publication et mention de la délivrance du brevet: **06.05.1999**
(21) Numéro de dépôt: 95925041.6
(22) Date de dépôt: 11.07.1995
(51) Int. Cl.: A61B 5/0484

(54) **DISPOSITIF D'EXAMEN D'UN SUJET, NOTAMMENT DE SES POTENTIELS EVOQUES VESTIBULAIRES**
UNTERSUCHUNGSGERÄT FÜR EINE PERSON BESONDERS VON VESTIBULAREN EVOZIERTEN POTENTIALEN
DEVICE FOR EXAMINING A PATIENT AND, IN PARTICULAR, DETERMINING HIS OR HER VESTIBULAR EVOKED POTENTIALS

(30) Priorité: 15.07.1994 FR 9408816
(43) Date de publication de la demande: 07.05.1997
(73) Titulaire: CENTRE NATIONAL DE LA RECHERCHE SCIENTIFIQUE (CNRS), 75794 Paris Cédex 16 (FR)
(72) Inventeur: BAUDONNIERE, Pierre-Marie, F-75015 Paris (FR); JOUEN, François, F-75014 Paris (FR); LEPECQ, Jean-Claude, F-54000 Nancy (FR); RENAULT, Bernard, F-75013 Paris (FR)
(74) Mandataire: Plaçais, Jean-Yves
(86) Numéro de dépôt international: FR9500931
(87) Numéro de publication internationale: WO9602186

(56) Documents cités:
- WO-A-83/03341
- ACTA OTOLARYNGOL, vol. 102, 1986 STOCKH., SE, pages 175-178, R.KAST ET AL 'Otolithic Evoked Potentials: A New Technique for Vestibular Studies'
- ELECTROENCEPHALOGRAPHY AND CLINICAL NEUROPHYSIOLOGY, vol. 80, no. 2, Mars 1991 IRELAND, pages 140-145, J.ELIDAN ET AL 'Short and middle latency vestibular evoked responses to acceleration in man'
- MEDICAL PROGRESS THROUGH TECHNOLOGY, vol. 20, no. 1-2, Janvier 1994 AMSTERDAM,NL, pages 31-35, CAUPOLICAN MUNOZ-GAMBOA ET AL 'Human vestibular evoked responses'

## Description

L'invention concerne un dispositif d'examen d'un sujet, notamment de ses potentiels évoqués vestibulaires par stimulation de son système vestibulaire.

On entend par sujet, tout être vivant pourvu notamment d'un système vestibulaire, qu'il soit cliniquement malade, et dans ce cas il s'agit d'un patient, ou bien qu'il soit cliniquement sain.

Le système vestibulaire peut être schématiquement considéré comme un système d'accéléromètres permettant au système nerveux central d'un sujet de mesurer la résultante à la fois de la gravité et des accélérations dans les trois plans définissant l'espace.

Ce système vestibulaire se compose de deux sous-systèmes fonctionnels distincts, mais complémentaires. Le premier sous-système dit canalaire comprend les canaux dits semi-circulaires, lesquels sont clairement dévolus à la mesure des accélérations angulaires du corps. Le second sous-système dit otolithique se divise en deux parties qui assurent deux fonctions complémentaires : le système utriculaire et le système sacculaire. La première fonction assurée concerne l'analyse des variations des forces de pesanteur liées à la gravité, et la seconde fonction est une mesure des accélérations linéaires dans les trois plans qui définissent l'espace (plan X (sagittal), plan Y (latéral), et plan Z (vertical)), comme l'ont démontré HIXSON, W.C.; NIVERI, J.J.; CORRELA, M.J., dans leur article "Kinematics nomenclature for physiological accelerations with special reference to vestibular applications", Monograph 14, Naval Aerospace Medical Institute, Naval Aerospace Medical Center, Pensacola, FL, 1966.

Le système vestibulaire étant en grande partie responsable de l'équilibre d'un être vivant, et notamment d'un homme, il est nécessaire de pouvoir tester spécifiquement son fonctionnement, soit pour vérifier son intégrité, soit pour en détecter les diverses pathologies et/ou mesurer les effets des thérapies vestibulaires.

Un tel test peut être réalisé en stimulant spécifiquement le système vestibulaire.

En effet, la stimulation du système vestibulaire, comme celle de nombreux autres parties du système nerveux, génère des ondes d'activité cérébrales provenant de régions identifiables du cerveau, lesquelles ondes, appelées potentiels évoqués, peuvent être recueillies notamment au niveau du scalp d'un individu.

Il existe ainsi des potentiels évoqués visuels, auditifs ou somesthésiques, qui sont quotidiennement utilisés en neurologie, parce que leur origine est relativement bien connue. En revanche, l'origine des potentiels évoqués vestibulaires (PEVest) est très mal connue.

La raison principale de cette méconnaissance des zones cérébrales génératrices des PEVest réside dans l'anatomo-physiologie du système vestibulaire qui est contrôlé par le système nerveux central dans sa partie sous-corticale. En effet, les neurones vestibulaires dits de second ordre sont déjà des centres de traitement et d'intégration des stimulations issues du système vestibulaire périphérique. Ces neurones de second ordre sont dits poly-afférentés, car ils sont en liaison avec un grand nombre de structures nerveuses, lesquelles sont impliquées dans la régulation non consciente de la posture. En conséquence, il est difficile de déterminer précisément quelles sont les zones de projection corticale du système vestibulaire.

Jusqu'à présent, les recherches effectuées sur le système vestibulaire ont essentiellement porté sur les potentiels évoqués vestibulaires liés à la stimulation du premier sous-système comprenant les canaux semi-circulaires. Ces recherches ont été rendues possibles par la mise au point de dispositifs permettant de déplacer en rotation le corps d'un sujet. Certaines zones de projection vestibulaire impliquées dans les sensations de rotation ont ainsi pu être mises en évidence, grâce à de tels dispositifs.

Si les potentiels évoqués d'origine canalaire sont relativement bien documentés, en revanche, les potentiels évoqués d'origine otolithique (second sous-système vestibulaire), induits par une accélération linéaire, ne sont pour ainsi dire pas connus. Jusqu'à présent, seule une expérience a donné des résultats. Celle-ci a été réalisée par KAST et LANKFORD., Elle est décrite et analysée dans l'article : KAST, R. et LANKFORD, J.E., "Otolithic evoked potentials : a new technique for vestibular studies", 1986, Acta Otolaryngol., 102, 175-178.

Le dispositif de KAST et LANKFORD comprend :
- un bâti logeant un équipage, mobile dans au moins une direction à l'intérieur dudit bâti et apte à supporter et immobiliser un sujet,
- des moyens d'entraînement aptes à déplacer ledit équipage dans au moins une direction, pour stimuler le système vestibulaire du sujet,
- un casque d'électro-physiologie adaptable sur le scalp dudit sujet, et muni de plusieurs électrodes, en positions respectives choisies, chaque électrode étant apte à détecter des ondes d'activité cérébrale provenant de régions identifiables du cerveau,
- des moyens de pilotage mécanique desdits moyens d'entraînement, en fonction d'une loi de mouvement choisie,
- ainsi que des moyens de traitement aptes à recueillir les ondes détectées par lesdites électrodes, afin d'en tirer des potentiels évoqués, et de rechercher de tels potentiels évoqués qui soient en relation avec la loi de mouvement choisie.

Dans un tel dispositif, l'être vivant qui est un homme est allongé sur une table légèrement inclinée. La stimulation vestibulaire, et notamment otolithique, est obtenue par une chute de la tête suivant un axe vertical par rapport au sol. Le casque d'électro-physiologie comprend quatre électrodes. Par ailleurs, les sujets examinés sont tous normaux (sans pathologie neurologique ou vestibulaire attestée).

Un tel dispositif présente un certain nombre d'inconvénients.

Un premier inconvénient réside dans le fait que la stimulation otolithique n'est pas strictement linéaire, puisque le sujet est allongé sur un plan incliné, ce qui implique que la stimulation utilisée combine à la fois une translation et une rotation.

Un second inconvénient réside dans le fait que les résultats ont été obtenus sur des sujets normaux, ce qui implique que lesdits résultats n'ont fait l'objet d'aucune validation par l'examen de sujets souffrant d'atteinte otolithique.

Un troisième inconvénient réside dans le fait que la couverture électro-encéphalographique proposée par le casque d'électrophysiologie est particulièrement pauvre et ne permet pas une analyse topographique précise des parties du cerveau impliquées dans la réponse vestibulaire.

Un quatrième inconvénient réside dans le fait que les afférences visuelles, auditives et somesthésiques n'étaient pas particulièrement bien contrôlées et pouvaient donc parasiter les potentiels évoqués vestibulaires étudiés.

On entend par afférences, les informations véhiculées par des voies neuronales périphériques en direction du système nerveux central. Ces informations issues de différentes parties du corps, viennent se superposer aux informations qui proviennent du système vestibulaire du sujet. Ainsi, l'installation d'un sujet dans un fauteuil mal adapté, dans lequel il ne se sent pas à l'aise, constitue une afférence somesthésique parasite.

Un cinquième inconvénient, et non le moindre, réside dans le fait que, d'une part, la durée des stimulations n'est pas suffisamment courte, ce qui fait qu'elles ne peuvent pas être considérées comme ponctuelles, et d'autre part, le profil de stimulation est sensiblement sinusoïdal, le sujet étant soumis à des accélérations variables suivies de décélérations variables, sans période de repos intermédiaire. Dans de telles conditions, il est particulièrement difficile d'isoler une réponse spécifique du système otolithique.

Enfin, un dernier inconvénient réside dans le fait que le dispositif mis au point par KAST et LANKFORD ne permettait de soumettre le sujet qu'à un type, et un seul, de profil de stimulation (sinusoïdal).

La présente invention a pour but d'améliorer la situation, en apportant des solutions à ces problèmes.

Un premier but de l'invention est de permettre soit une étude spécifique du premier sous-système dit canalaire ou du second sous-système dit otolithique, soit une étude couplée de ces deux sous-systèmes.

Un second but de l'invention est de proposer de nombreux profils de stimulation différents et adaptables à la pathologie du sujet à examiner.

Un troisième but de l'invention est de notablement améliorer la sensibilité de détection des zones cérébrales impliquées dans la projection vestibulaire.

Un quatrième but de l'invention est de mieux s'assurer de l'origine vestibulaire de la stimulation, en contôlant les afférences auditives, visuelles, et somesthésiques parasites sur le sujet.

Un cinquième but de l'invention est de proposer un dispositif comprenant des moyens de traitement aptes à analyser et traiter les potentiels évoqués vestibulaires en temps réel.

Enfin, un sixième but de l'invention est de mettre à disposition, dans un même dispositif, et sous forme automatisée, un ensemble d'examens prédéfinis, ainsi qu'une base de données comprenant des examens tests autorisant des comparaisons avec un ou plusieurs examens précédemment effectués.

L'invention propose à cet effet un dispositif de test sur un sujet, du type défini précédemment, et dans lequel les moyens d'entraînement sont agencés pour permettre un déplacement en translation suivant au moins l'une des trois directions définissant l'espace, et un déplacement en rotation suivant un des plans définissant ledit espace, dudit équipage,
dans lequel les moyens de pilotage mécanique desdits moyens d'entraînement, sont agencés pour permettre l'application au sujet d'une stimulation élémentaire en déplacement de caractéristiques spatio/temporelles monotones, avec un profil ajustable en vitesse et/ou accélération, dans au moins une desdites trois directions de l'espace en translation et/ou dans au moins un desdits trois plans de l'espace en rotation,
et dans lequel les moyens de traitement sont aptes à numériser les ondes détectées par certaines, au moins deux, desdites électrodes, et sont agencés pour y rechercher des potentiels évoqués en rapport avec le profil de la stimulation élémentaire.

Cela permet d'obtenir des informations en temps réel sur le fonctionnement d'au moins une partie du système vestibulaire, et plus spécifiquement soit du sous-système canalaire, soit du sous-système otolithique, soit encore d'une combinaison de ces deux sous-systèmes.

Selon une autre caractéristique de l'invention, les moyens de traitement sont en outre aptes à mémoriser des caractéristiques spatio-temporelles d'examen, chaque examen étant défini par la répétition d'au moins un cycle de stimulation d'au moins une phase de stimulation élémentaire choisie parmi une première phase dans laquelle l'équipage est soumis à une accélération dans une direction ou un sens de déplacement donné(e), une seconde phase dans laquelle l'équipage est soumis à une vitesse de translation ou de rotation constante dans cette même direction ou ce même sens de déplacement, et une troisième phase dans laquelle l'équipage est soumis à une décélération dans une direction ou un sens de déplacement opposé(e) à celle ou à celui des première et seconde phases.

Ainsi, l'invention permet de soumettre un être vivant à des accélérations ou décélérations de type linéaire et/ou rotatoires, sur commande. Mais elle permet également, lors des phases de vitesse constante, de ménager des périodes de non-stimulation pendant lesquelles le système vestibulaire de l'être vivant n'est pas sollicité.

Dans une forme de réalisation de l'invention, l'accélération de la première phase est d'intensité comprise entre 0,1 g et 3 g, et la durée de cette première phase est comprise entre 1 milliseconde et 100 millisecondes. La vitesse constante de la seconde phase dépend de la vitesse de l'équipage à la fin de la première phase, et la durée de cette seconde phase est comprise entre 0 seconde et 5 secondes, et l'accélération de la troisième phase est d'intensité comprise entre 0,1 g et 3 g, et la durée de cette troisième phase est comprise entre 1 milliseconde et 100 millisecondes.

De telles caractéristiques permettent de stimuler un être vivant, et notamment un homme, sans pour autant que son système vestibulaire soit saturé, ce qui est en général le cas au-delà d'accélérations d'intensité supérieure à 3 g. Par ailleurs, la durée des accélérations est choisie de telle sorte que la stimulation soit considérée comme quasi-ponctuelle, ce qui permet de bien discerner la réponse du système vestibulaire à une telle stimulation.

Selon encore une autre caractéristique de l'invention, les moyens de traitement sont également aptes à valider un cycle en fonction de critères d'admissibilité sur le mouvement des yeux, la forme et/ou l'amplitude des ondes détectées par les électrodes.

Ainsi, les moyens de traitement sont aptes à autoriser un n-ième cycle après validation du (n-1)-ième cycle, ainsi qu'à recommencer un n-ième cycle jusqu'à sa validation.

De la sorte, chaque cycle retenu peut être considéré comme un cycle exploitable.

Avantageusement, après validation de chaque cycle, les moyens de traitement sont aptes à délivrer sur un moniteur vidéo au moins une image de type tableau de courbes de potentiels évoqués représentatives de l'amplitude de l'émission des régions du cerveau détectée par chaque électrode en réponse à chaque phase de stimulation du cycle.

Préférentiellement, l'image délivrée après un n-ième cycle, d'une succession de cycles identiques, est représentative de la moyenne des amplitudes détectées au cours des n cycles identiques écoulés.

Ainsi, lorsque les cycles d'un examen sont tous identiques, l'image qui s'affiche sur l'écran au fur et à mesure que les cycles s'écoulent, constitue une consolidation de l'ensemble des images associées aux cycles précédents.

Il devient alors possible, à la personne qui commande le dispositif (en général un spécialiste), d'interrompre à tout moment l'examen en cours, lorsqu'il juge que la réponse aux stimulations successives n'évolue plus notablement.

Cependant, puisque les cycles d'un examen peuvent être différents, répétés ou non, successifs ou non, en cas de cycles différents les uns des autres, l'image correspondant au n-ième cycle est représentative du seul cycle numéro n.

Selon encore une autre caractéristique de l'invention, les moyens de traitement comprennent également une mémoire de masse apte à stocker dans un fichier l'ensemble des données résultant de chaque cycle d'un examen, lesquelles données forment résultats d'examen. Cela permet d'analyser ultérieurement les résultats d'un examen, cycle après cycle, ou même phase après phase.

A cet effet, les moyens de traitement sont également aptes à traiter les données contenues dans au moins un fichier d'examen, pour les transformer en données de champs de potentiel ou de densité de courant, et délivrer sur le moniteur vidéo au moins une image , de type représentation tridimensionnelle "3D" de la surface d'une calotte crânienne d'être vivant, représentative des données résultant d'au moins un n-ième cycle de l'examen.

Avantageusement, la mémoire de masse est en outre apte à stocker des données relatives à des examens tests formant base de données, pour autoriser au moins une comparaison, sur le moniteur vidéo, entre les résultats d'au moins un examen, ou d'au moins un n-ième cycle d'au moins un examen, et les données d'au moins un examen test.

Il est désormais possible d'effectuer des comparaisons entre sujets atteints de pathologie et sujets normaux, ou bien encore entre une pluralité de sujets atteints de pathologies différentes ou identiques.

Dans le mode de réalisation préférentiel de l'invention, l'équipage comprend en outre un appuie-tête apte à interdire tout mouvement de tête lors d'un examen, un générateur de bruit blanc apte à contrôler des informations auditives parasites, ainsi qu'un cache visuel apte à contrôler temporairement des informations visuelles, ce qui permet de mieux s'assurer de l'origine vestibulaire de la stimulation. Par ailleurs, le dispositif comprend au moins deux électrodes aptes à détecter les mouvements horizontaux et verticaux des yeux, et les moyens de traitement comprennent également un module d'amplification des signaux détectés par les électrodes.

Ainsi, l'être vivant stimulé est isolé du monde extérieur, et notamment des stimulations auditives et visuelles pouvant interférer avec les informations vestibulaires.

D'autres caractéristiques et avantages de l'invention apparaîtront à l'examen de la description détaillée ci-après, et des dessins annexés, sur lesquels :
- la figure 1 est un schéma en vue latérale du bâti et de l'équipage mobile dans une forme de réalisation de l'invention;
- la figure 2 est une vue du dessus du bâti et de l'équipage mobile dans une forme de réalisation de l'invention;
- la figure 3 est une vue du dessus du casque d'électro-physiologie illustrant la position des électrode, en référence à un crâne;
- la figure 4 décrit les relations entre les principaux éléments du dispositif selon l'invention;
- la figure 5 est un organigramme illustrant les étapes successives d'un examen type;
- la figure 6 est une copie d'écran illustrant un tableau de courbes de potentiels évoqués otolithiques, à l'intérieur d'une fenêtre de visualisation; et
- la figure 7 est une copie d'écran illustrant un crâne avec en fausses couleurs des champs de potentiels issus du traitement des résultats d'un examen, en référence à une représentation tridimensionnelle "3D" d'un crâne d'humain équipé d'électrodes.

Les dessins annexés sont, pour l'essentiel, de caractère certain. En conséquence, ils font partie intégrante de la description et pourront non seulement servir à compléter celle-ci, mais aussi contribuer à la définition de l'invention le cas échéant.

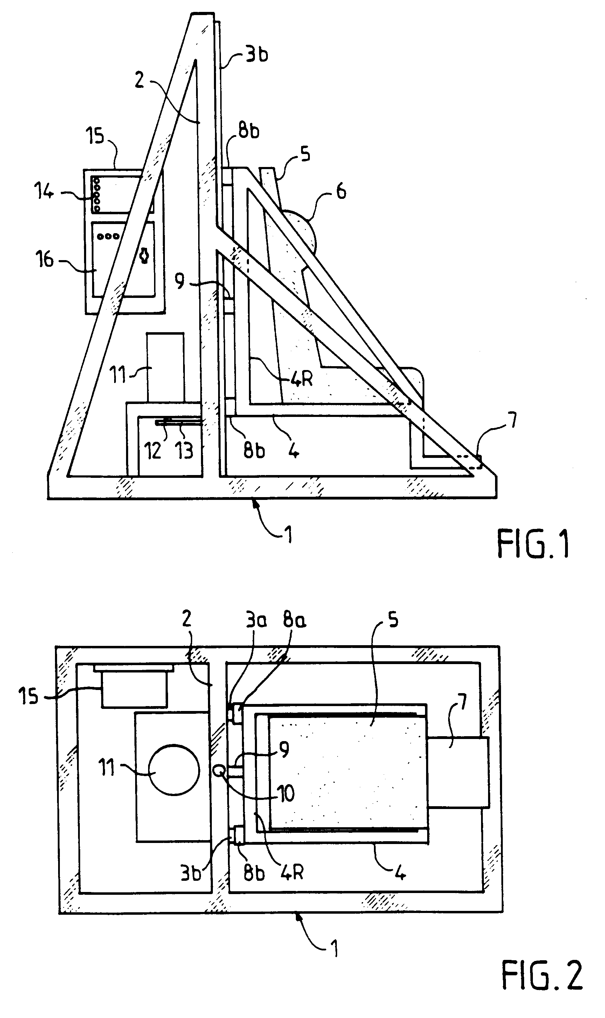

On se réfère tout d'abord aux figures 1 et 2.

Le dispositif a pour but de stimuler le système vestibulaire d'un être vivant, notamment d'un homme, de façon automatisée, et de traiter et analyser les données résultant de cette stimulation.

Dans ce but, le dispositif est constitué de deux parties distinctes, connectées l'une à l'autre : une partie mécanique pour stimuler le sujet, et une seconde partie apte, d'une première part, à assurer la commande de la première partie, d'une seconde part, à acquérir des signaux sur le scalp du sujet, et d'une troisième part, à traiter les signaux ainsi reçus.

La première partie, qui est essentiellement mécanique, comprend un bâti 1, réalisé par assemblage de panneaux et d'éléments rigides et massifs, autorisant de brusques déplacements d'un équipage mobile 4 pouvant supporter des masses d'environ 200 kg, à l'intérieur de sa structure.

Dans la forme de réalisation décrite, le bâti 1 comprend un panneau vertical 2 sur lequel sont fixés deux éléments 3 également verticaux formant rails de guidage pour un équipage 4 mobile.

Cet équipage mobile 4 loge un fauteuil 5, de type baquet, équipé d'un harnais (non représenté sur la figure), apte à maintenir immobile le corps du sujet pendant la durée d'un examen.

Ce fauteuil 5 comprend également un appuie-tête 6 propre à immobiliser la tête du sujet pour éviter tout mouvement latéral.

L'équipage mobile 4 comprend en outre un repose-pieds 7 de hauteur réglable.

Ainsi, un sujet installé dans le fauteuil 5 ergonomique, ses pieds reposant sur le repose-pieds 7, n'est pas soumis à des afférences somesthésiques parasites.

Par ailleurs, la structure formant l'équipage mobile 4 comprend dans sa partie arrière un cadre métallique 4R, de forme sensiblement rectangulaire, et muni en chacun de ses coins d'une glissière 8a ou 8b apte à coulisser à l'intérieur de l'un des rails de guidage 3.

Ainsi, les deux glissières 8a peuvent coulisser dans le rail de guidage 3a et, dans le même temps, les deux glissières 8b peuvent coulisser à l'intérieur du rail 3b, entraînant avec elles l'équipage mobile 4.

Enfin, le cadre métallique de l'équipage mobile comprend des moyens de fixation 9 aptes à faire coulisser ledit équipage sur une tige filetée 10 verticale formant vis sans fin, et logée dans un conduit vertical centré à l'intérieur du panneau vertical 2 du bâti 1.

Cette vis sans fin 10 est entraînée en rotation par des moyens d'entraînement comprenant un moteur pas à pas 11 muni d'un axe de sortie 12. Cet axe 12 est muni en son extrémité d'un pignon (non représenté sur les figures) apte à entraîner une courroie crantée 13 qui entoure également un second pignon (non représenté sur les figures) solidaire de la vis sans fin 10.

Ainsi, lorsque l'axe 12 du moteur 11 est en rotation, cela entraîne la courroie 13, laquelle communique le mouvement au pignon solidaire de la vis sans fin 10, ce qui permet de faire monter ou descendre l'équipage mobile 4 dans le bâti 1.

Le moteur 11 est alimenté par une unité de puissance 14 logée dans une armoire 15 fixée sur la structure du bâti 1. Cette armoire 15 comprend également une interface 16 de connexion à des moyens de pilotage mécanique qui seront décrits ultérieurement.

La seconde partie du dispositif comprend un ordinateur apte à piloter le moteur 11, ainsi qu'à traiter et analyser des données issues d'un casque d'électro-physiologie 17 adaptable sur le scalp d'un sujet installé dans le fauteuil 5.

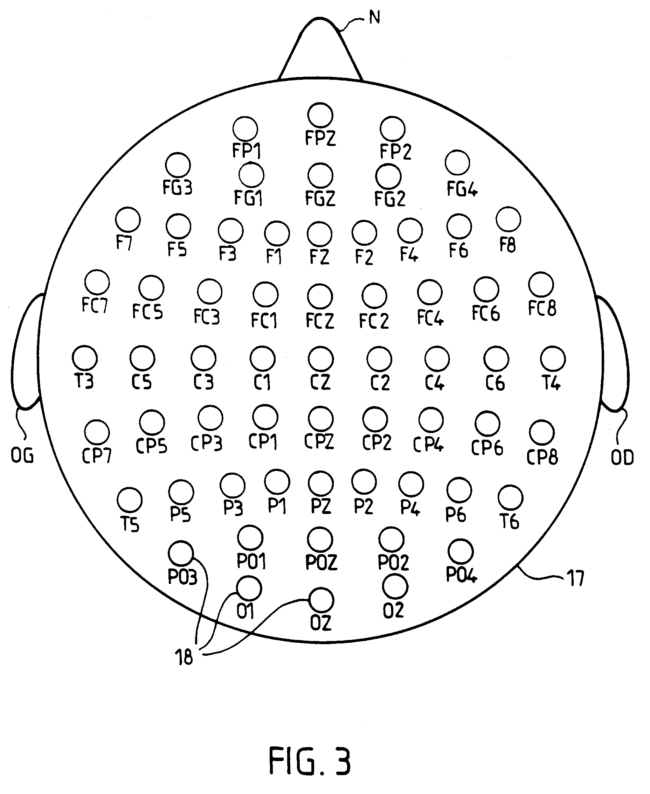

On se réfère maintenant à la figure 3 qui décrit le casque d'électrophysiologie 17.

Ce casque 17 comprend une pluralité d'électrodes 18. Selon la précision de l'examen, ce casque peut comporter soit 10 électrodes, soit 32 électrodes, soit encore 64 électrodes. Dans ces trois cas (10, 32, 64 électrodes), le casque d'électro-physiologie 17 répond à la norme internationale dite 10/20.

A titre de comparaison, le casque utilisé par KAST et LANKFORD ne comportait que quatre électrodes utilisées en position dite "AEP", à savoir: une électrode de mise à la masse ("Forehead"), une électrode de référence ("Vertex"), et deux électrodes actives ("Mastoïde droite et gauche").

Le casque est réalisé dans un matériau souple, et sa forme est sensiblement sphérique, ce qui permet de l'adapter sur le crâne d'un sujet de type humain.

Néanmoins, tout autre casque ou montage équipé d'électrodes aptes à détecter des ondes d'activité cérébrale sur le scalp d'un sujet pourra être utilisé.

Sur la figure 3 sont représentées les positions respective des 32 électrodes en référence aux oreilles droite OD et gauche OG, ainsi qu'au nez N d'un sujet de type humain. Chaque électrode définit une zone cérébrale sur le scalp du sujet, chaque zone cérébrale étant associée à au moins une région identifiable du cerveau, supposée être une projection vestibulaire.

Ces électrodes 18 sont aptes à détecter des ondes d'activité cérébrale émises par une ou des régions du cerveau , en réponse à une stimulation du système vestibulaire, laquelle stimulation est générée par la translation verticale de l'équipage mobile 4 dans le bâti 1.

Les ondes d'activité cérébrale détectées par les électrodes 18 seront recueillies par des moyens de traitement et d'analyse 23, afin d'en tirer des potentiels évoqués vestibulaires, et de rechercher de tels potentiels évoqués qui soient en relation avec la loi de mouvement choisie pour stimuler le système vestibulaire du sujet.

Afin de déterminer avec un maximum de précision les régions du cerveau impliquées dans la réponse vestibulaire à la stimulation, on pourra effectuer une analyse des potentiels évoqués par combinaison linéaire de certaines électrodes 18 du casque 17.

Par ailleurs, de façon à contrôler au mieux l'origine vestibulaire de la stimulation, deux dispositifs annexes (non représentés sur les figures) sont joints au fauteuil 5. Le premier dispositif annexe est un générateur de bruit blanc qui permet de supprimer les informations auditives parasites. Le second dispositif annexe est un système permettant de supprimer temporairement les informations visuelles.

Enfin, le dispositif comprend également au moins deux électrodes (non représentées sur les figures) destinées à être fixées au voisinage des paupières du sujet afin de recueillir les mouvements verticaux et horizontaux de ses yeux.

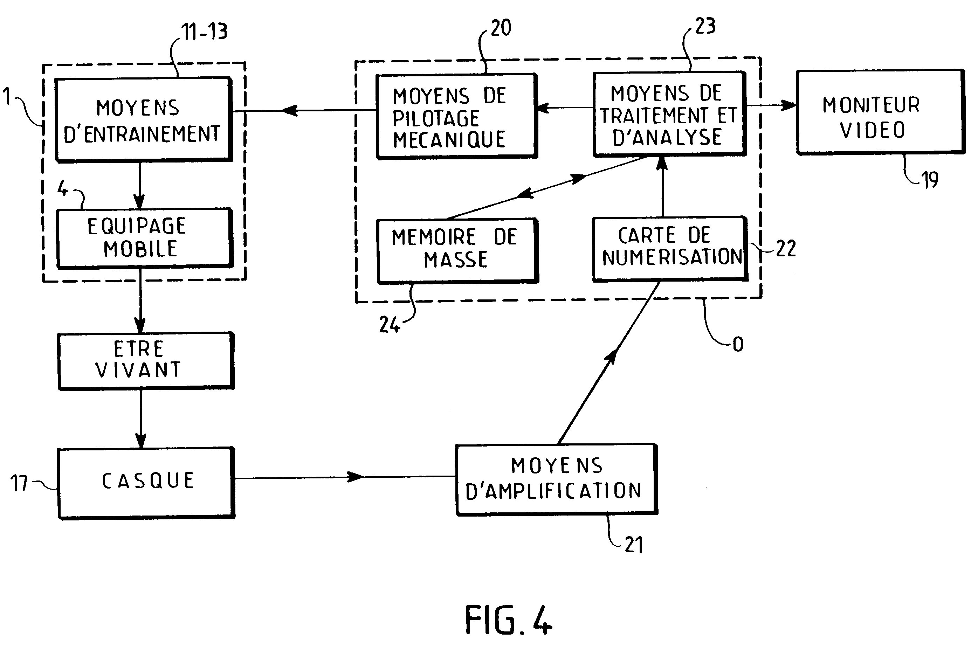

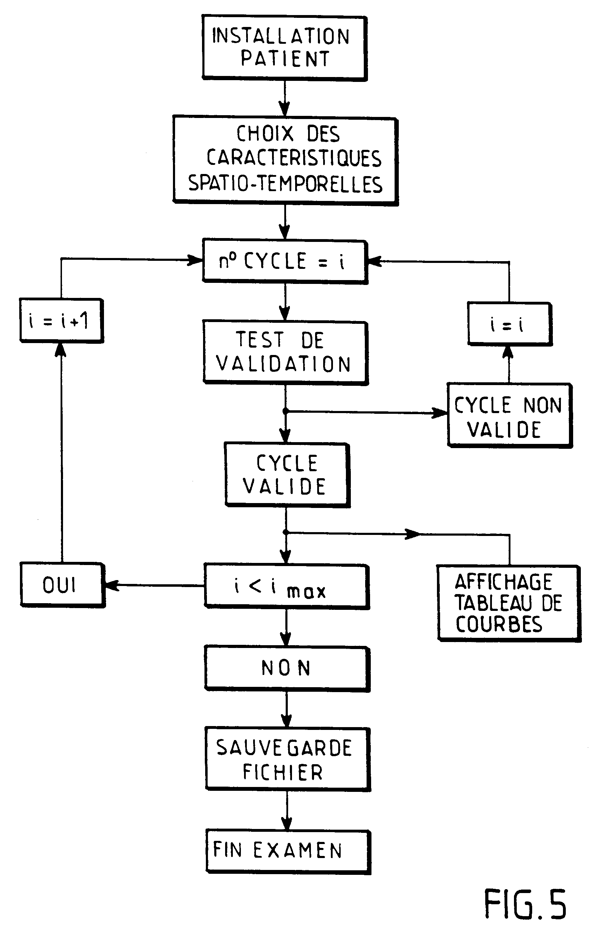

On se réfère maintenant aux figures 4 et 5, pour décrire les connexions entre les différents éléments du dispositif ainsi que les principales étapes d'un examen.

Lorsqu'un spécialiste décide de soumettre un sujet à un examen vestibulaire, il l'équipe dans un premier temps du casque 17, du générateur de bruit blanc, du système de suppression des informations visuelles, ainsi qu'au moins deux électrodes destinées à recueillir les mouvements oculaires.

Le sujet est ensuite installé dans le fauteuil 5 de l'équipage mobile 4. On l'immobilise ensuite dans ce fauteuil 5 grâce au harnais adaptable, ainsi qu'à l'appuie-tête 6. La hauteur du repose-pieds 7 peut alors être réglée en fonction de la taille du sujet. L'examen peut alors commencer.

Le spécialiste est installé devant la console de son ordinateur O. Sur l'écran vidéo 19 de l'ordinateur O, s'affiche un menu qui lui permet de sélectionner les caractéristiques spatio-temporelles monotones de l'examen qu'il veut effectuer.

On entend par déplacement de caractéritique monotone, tout déplacement effectué dans une même direction au cours d'une période déterminée correspondant à une stimulation élémentaire. En d'autre termes, cela correspond à une vitesse et/ou une accélération, soit positives ou nulles, soit négatives ou nulles (décélération).

Un examen vestibulaire est constitué de la répétition d'au moins un cycle de stimulation. Un cycle de stimulation comprend au moins une phase de stimulation dite élémentaire choisie parmi trois phases :
- une première phase dans laquelle l'équipage est soumis à une accélération dans une direction de déplacement donnée, en l'occurrence verticale,
- une seconde phase dans laquelle l'équipage est soumis à une vitesse de translation constante dans cette même direction de déplacement vertical, et
- une troisième phase dans laquelle l'équipage est soumis à une décélération dans la même direction de déplacement que celle des première et seconde phases.

Le profil de la stimulation est donc de forme quelconque, comme par exemple trapézoïdale, triangulaire, ou sinusoïdale.

Par ailleurs, le sens de déplacement linéaire d'un examen peut être choisi vers le haut (accélération positive), puis vers le bas (accélération négative), ou bien seulement vers le haut ou seulement vers le bas.

Le spécialiste choisit ainsi les caractéristiques spatio-temporelles de chaque phase de chaque cycle de l'examen.

En général, un examen est constitué par la répétition de n cycles identiques de trois phases.

Les caractéristiques spatio-temporelles sont la direction de translation, le nombre de répétitions et de phases de chaque cycle, la durée de chacune des phases de chacun desdits cycles, et l'intensité de l'accélération de chaque première et/ou troisième phase d'un cycle.

La vitesse de la seconde phase est celle de l'équipage 4 à la fin de la première phase d'accélération. Elle est donc fonction non seulement de l'intensité de l'accélération, mais également de sa durée.

Dans la forme de réalisation proposée, le moteur 11 est apte a soumettre l'équipage mobile à des accélérations monotones verticales d'intensité comprise entre 0,1 g et 3 g pendant des durées comprises entre 1 milliseconde et 100 millisecondes. Ce moteur 11 peut également soumettre l'équipage mobile 4 à une vitesse constante pendant une durée comprise entre 0 seconde et 5 secondes.

Par ailleurs, la durée des phases d'accélération a été choisie de telle sorte que, d'une part, la stimulation soit considérée par le système vestibulaire comme quasi-ponctuelle, et d'autre part, que l'on puisse isoler les réponses vestibulaires à cette stimulation.

Une fois l'ensemble des caractéristiques spatio-temporelles saisies par le spécialiste, l'ordinateur O est apte à piloter le moteur 11. Pour cela, il comprend des moyens de pilotage mécanique 20 qui sont reliés par un câble de liaison à l'interface 16 logée dans l'armoire 15 du bâti 1.

Ainsi, sur ordre des moyens de pilotage mécanique 20, le moteur 11 va provoquer le déplacement de l'équipage 4 selon les caractéristiques spatio-temporelles saisies par le spécialiste.

Ainsi stimulé, le système vestibulaire du sujet, et notamment son système otolithique puisqu'il s'agit dans l'exemple choisi d'une stimulation de type accélération verticale, répond en émettant au niveau de certaines régions du cerveau, des ondes d'activité cérébrale qui vont être transmises au niveau du scalp, puis recueillies par les électrodes 18 du casque 17.

Ce casque 17 est connecté à des moyens d'amplification 21, lesquels sont connectés à une carte de numérisation 22 logée dans l'ordinateur O.

Les données recueillies par les électrodes 18 sont des différences de potentiel dont l'amplitude dépend, d'une part, du nombre de neurones impliqués dans la réponse, et d'autre part, de la profondeur à laquelle se trouve la partie du cerveau qui émet.

Ces données vont être numérisées par la carte de numérisation 22, puis traitées en temps réel par les moyens de traitement et d'analyse 23 de l'ordinateur O. Les données résultant de ce traitement forment résultats d'examen. Elles seront ensuite affichés sur l'écran vidéo 19 de l'ordinateur, à l'intérieur d'une fenêtre, sous la forme d'un tableau de courbes de potentiels évoqués vestibulaires et notamment otolithiques, chaque courbe étant représentative du signal reçu par une électrode 18 du casque 17.

Les données résultant du traitement par les moyens de traitement et d'analyse 23 sont également stockées sous forme de fichiers, préférentiellement de format texte, dans une mémoire de masse 24 de l'ordinateur O.

La procédure détaillée d'un examen est la suivante.

Après l'installation du sujet dans l'équipage mobile 4, et le choix des caractéristiques spatio-temporelles de l'examen, le spécialiste donne l'ordre à l'ordinateur de commencer l'examen. On se place dans un cas classique d'examen, à savoir un cycle unique répété n fois, chaque cycle étant constitué de trois phases, une première phase d'accélération, une seconde phase à vitesse constante, et une troisième phase de décélération.

Sur ordre des moyens de commande 20 dez l'ordinateur O, le moteur 11 se met en action, et communique une accélération à l'équipage mobile 4, ce qui provoque sa translation vers le haut. L'intensité de l'accélération de cette première phase sera par exemple de 0,3 g, et la durée de cette première phase est d'environ 30 millisecondes.

Stimule par une telle accélération, le système vestibulaire, et notamment le sous-système otolithique du sujet, répond en émettant au niveau de certaines régions du cerveau des ondes d'activité cérébrale. Ces ondes sont détectées par les électrodes 18 du casque d'électrophysiologie 17.

Chaque électrode 18 interprète la ou les ondes ainsi détectées au niveau du scalp en tant que différence de potentiel d'amplitude donnée. Ces 32 différences de potentiels détectées sont ensuite amplifiées par les moyens d'amplification 21 qui comportent 32 voies d'entrée et 32 voies de sortie, puis acheminées au niveau de la carte de numérisation 22. Une fois numérisés, les 32 signaux sont traités par les moyens de traitement et d'analyse 23, lesquels soumettent l'ensemble des potentiels ainsi traités à des critères d'admissibilité. Ces critères constituent des tests de validité pour la phase du cycle en cours.

Les deux critères sont, d'une part, le non-parasitage des mesures par une afférence visuelle détecté par l'analyse des mouvements des yeux, laquelle est effectuée par les deux électrodes précédemment décrites, et d'autre part, par un test d'écrêtage du potentiel au niveau des électrodes. Les critères de rejet sont effectués en temps réel. Ainsi, le cycle écoulé est soit validé, soit invalidé.

En cas de non-validation de ce premier cycle, les moyens de traitement et d'analyse 23 sont aptes à ordonner au moteur 11 de recommencer intégralement le cycle précédemment effectué. Il va de soi que ce premier cycle sera recommencé autant de fois que les moyens de traitement et d'analyse 23 le jugeront nécessaires.

En revanche, en cas de validation du premier cycle, les moyens de traitement et d'analyse 23 sont aptes, d'une part à afficher sur le moniteur vidéo 19 un tableau de 32 courbes de potentiels évoqués représentatives des ondes détectées par les 32 électrodes du casque 17, et d'autre part à ordonner au moteur 11 d'effectuer le cycle numéro 2.

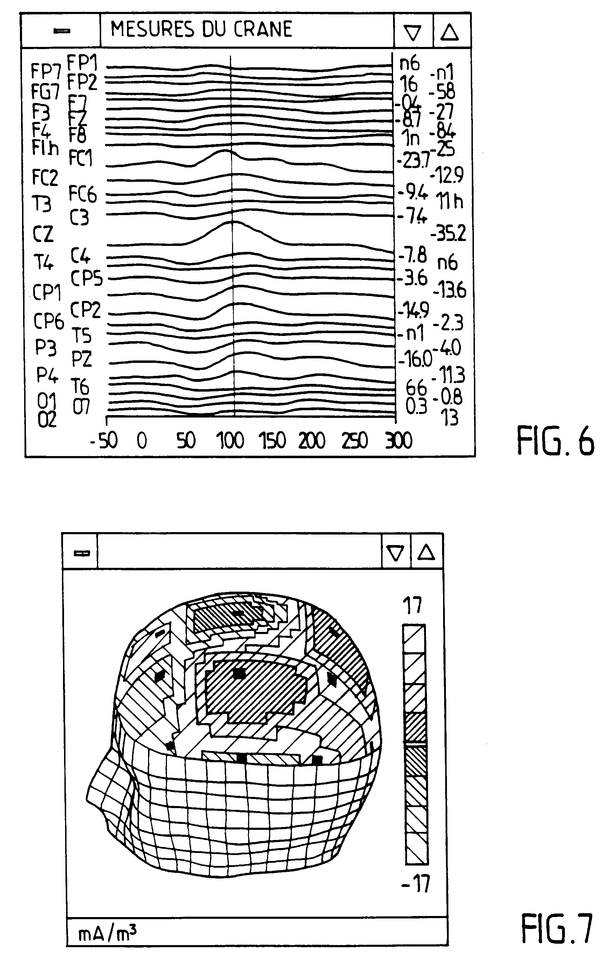

Un tel tableau est illustré sur la figure 6, sur laquelle l'axe des abscisses représente le temps en millisecondes, et l'axe des ordonnées représente l'amplitude du potentiel évoqué par rapport au zéro de chaque courbe. L'axe gauche des ordonnées est en outre utilisé pour la désignation des différentes électrodes, et l'axe droit des ordonnées est également utilisé pour indiquer l'amplitude maximale de chaque potentiel évoqué.

Cette procédure est réitérée jusqu'au n-ième cycle. A la fin de ce dernier cycle, l'ensemble des données est stocké sous forme de fichier d'examen dans la mémoire de masse 24 de l'ordinateur O.

Dans un tel fichier d'examen, les données sont stockées cycle après cycle.

A la fin d'un examen, le spécialiste a deux possibilités. Une première possibilité consiste à effectuer un autre examen, identique ou différent du précédent. Dans ce premier cas, il sélectionne les caractéristiques spatio-temporelles de l'examen à effectuer. La seconde possibilité consiste à analyser, immédiatement après l'examen, l'ensemble des données stockées dans le fichier d'examen. Dans ce cas, le spécialiste commande à l'ordinateur O de lui afficher sur l'écran vidéo 19 le tableau de courbes de potentiels évoqués et/ou une image tridimensionnelle "3D" représentant un crâne sur lequel est représentée, à l'aide d'un rectangle, la position des électrodes, avec en fausses couleurs autour desdites électrodes des zones représentatives de l'amplitude des potentiels évoqués détectés par chacune de ces électrodes.

Une telle image "3D" est illustrée sur la figure 7.

Les moyens de traitement et d'analyse 23 sont aptes à délivrer une image du crâne sous un angle quelconque, et représentative, soit de champs de potentiels, soit de champs de densités de courant.

Ces champs de potentiels ou de densités de courant sont calculés par les moyens de traitement et d'analyse 23 à partir des potentiels évoqués détectés par les électrodes, en utilisant une méthode de calcul mathématique dite de "spline sphérique".

Cette méthode de spline sphérique a été initialement développée par J.PERNIER, et divulguée dans l'article: "F.PERRIN, J.PERNIER, O.BERTRAND, and J.F.ECHALLIER, "Spherical splines for scalp potential and current density mapping", Electroencephaligraphy and Clinical neurophysiology, 1989, 72, 184-187".

En fait, la méthode de calcul utilisée est une adaptation de la méthode des splines sphériques, laquelle ne s'applique qu'à des surfaces sensiblement sphérique, ce qui n'est généralement pas le cas du scalp d'un humain. Cette méthode a donc été développée afin de tenir compte de la non sphéricité de la boîte cranienne d'un humain. En conséquence elle est appelée méthode de spline quasi-sphérique.

Par ailleurs, l'ensemble du dispositif étant géré par un environnement Windows (Marque déposée), le spécialiste peut commander l'affichage d'une pluralité de fenêtres sur l'écran vidéo 19, chaque fenêtre contenant soit un tableau de courbes de potentiels évoqués, soit une image tridimensionnelle d'un crâne représentative de champs de potentiels ou de densités de courant.

Les moyens de traitement et d'analyse 23 sont également aptes à afficher à l'intérieur d'une fenêtre de l'écran vidéo 19, une image tridimensionnelle évolutive, représentative de l'évolution soit des champs de potentiels, soit des champs de densités de courant au cours des différents cycles de l'examen concerné. Ainsi, le spécialiste voit évoluer au cours du temps dans une fenêtre donnée l'activité des différentes zones cérébrales définies autour de chaque électrode.

Il est également possible d'afficher dans plusieurs fenêtres différentes, l'image "3D" d'un même crâne vu sous des angles différents, ou à des instants différents, ou bien même représentant soit des champs de potentiels, soit des champs de densités de courant. Cette dernière solution permet de mieux analyser dans certains cas la pathologie du sujet examiné car les champs de potentiels et les champs de densités de courant délivrent des informations généralement complémentaires.

Mais il est également possible d'afficher sur l'écran vidéo 19, à l'intérieur de fenêtres différentes, des résultats d'examens issus d'examens différents effectués soit sur le même sujet, soit sur des sujets différents. Enfin, la mémoire de masse 23 de l'ordinateur O contient également des fichiers d'examens tests formant base de données, lesquels sont représentatifs soit de pathologies connues, soit de sujets normaux. Une telle base de données permet ainsi de comparer sur l'écran vidéo 19 les résultats d'un examen effectué sur un sujet, aux données d'un examen test parfaitement connu.

De façon particulièrement avantageuse, le spécialiste peut jouer sur le nombre d'électrodes qui seront traitées par les moyens de traitement et d'analyse 23, puis affichées sur un tableau de courbes de potentiels évoqués, ou bien sur une image tridimensionnelle de crâne. Cela permet de faire des analyses ponctuelles de zones privilégiées indépendamment des autres zones.

Il est également possible de faire varier l'intensité des couleurs afin de jouer sur le contraste d'une image tridimensionnelle sur l'écran vidéo 19.

Dans la description qui vient d'être faite, il a été fait référence à un ordinateur O pour piloter l'ensemble du dispositif. En fait un micro-ordinateur, ou plus généralement tout moyen apte, d'une part, à commander le déplacement du dispositif, et d'autre part, à traiter en temps réel les informations acquises au niveau du scalp du sujet puis à les mémoriser pour autoriser leur analyse ultérieure et leur comparaison à d'autres informations, peut être utilisé en lieu et place d'un ordinateur.

L'invention telle que décrite ne saurait se limiter à ce mode de réalisation. On peut envisager de réaliser un dispositif muni de plusieurs moteurs aptes chacun à déplacer l'équipage mobile soit en translation dans une direction perpendiculaire à la direction verticale, comme par exemple d'avant en arrière ou bien latéralement, soit en rotation dans un plan parallèle au plan du sol.

Mais il est également envisageable de combiner une translation avec une rotation. Pour autoriser de tels déplacements, un bâti adapté serait nécessaire.

Par ailleurs, les moyens d'entraînement ne sont pas limités au mode de réalisation décrit précédemment. Tout autre mode d'entraînement apte à communiquer un mouvement à l'équipage mobile peut être envisagé.

Enfin, un tel dispositif de test peut être adapté à l'examen de tout être vivant. Cela implique que d'autres types de fauteuils et de casque d'électrophysiologie peuvent être utilisés sans pour autant sortir du cadre de l'invention.

## Revendications

1. Dispositif d'examen d'un sujet, notamment de ses potentiels évoqués vestibulaires, du type comprenant :
- un bâti (1) logeant un équipage (4), mobile dans au moins une direction à l'intérieur dudit bâti et apte à supporter et immobiliser un sujet,
- des moyens d'entraînement (9-11) aptes à déplacer ledit équipage (4) dans au moins une direction, pour stimuler le système vestibulaire du sujet,
- un casque d'électro-physiologie (17) adaptable sur le scalp dudit sujet, et muni de plusieurs électrodes (18), en positions respectives choisies, chaque électrode étant apte à détecter des ondes d'activité cérébrale provenant de régions identifiables du cerveau,
- des moyens de pilotage mécanique (20) desdits moyens d'entraînement, en fonction d'une loi de mouvement choisie,
- ainsi que des moyens de traitement (23) aptes à recueillir les ondes détectées par lesdites électrodes (18), afin d'en tirer des potentiels évoqués, et de rechercher de tels potentiels évoqués qui soient en relation avec la loi de mouvement choisie,
caractérisé en ce que les moyens d'entraînement (9-11) sont agencés pour permettre un déplacement en translation suivant au moins l'une des trois directions définissant l'espace, et/ou un déplacement en rotation suivant un des plans définissant ledit espace, dudit équipage (4),
en ce que les moyens de pilotage mécanique (20) desdits moyens d'entraînement, sont agencés pour permettre l'application au sujet d'une stimulation élémentaire en déplacement de caractéristiques spatio/temporelles monotones, avec un profil ajustable en vitesse et/ou accélération, dans au moins une desdites trois directions de l'espace en translation et/ou dans au moins un desdits trois plans de l'espace en rotation,
en ce que les moyens de traitement (23) sont aptes à numériser les ondes détectées par certaines, au moins deux, desdites électrodes (18), et sont agencés pour y rechercher des potentiels évoqués en rapport avec le profil de la stimulation élémentaire,
ce qui permet d'obtenir des informations en temps réel sur le fonctionnement d'une partie au moins du système vestibulaire.

2. Dispositif selon la revendication 1, caractérisé en ce que chaque donnée de potentiel évoqué est une amplitude, laquelle est transformable sur commande, par les moyens de traitement (23), en champ de potentiel ou en densité de courant.

3. Dispositif selon l'une des revendications 1 et 2, caractérisé en ce que les moyens de traitement (23) sont en outre aptes à mémoriser des caractéristiques spatio-temporelles d'examen, chaque examen étant défini par la répétition d'au moins un cycle de stimulation d'au moins une phase de stimulation élémentaire choisie parmi une première phase dans laquelle l'équipage (4) est soumis à une accélération dans une direction ou un sens de déplacement donné(e), une seconde phase dans laquelle l'équipage (4) est soumis à une vitesse de translation ou de rotation constante dans cette même direction ou ce même sens de déplacement, et une troisième phase dans laquelle l'équipage (4) est soumis à une décélération dans la même direction ou le même sens de déplacement que celle ou celui des première et seconde phases.

4. Dispositif selon la revendication 3, caractérisé en ce que les caractéristiques spatio-temporelles d'un examen sont la direction de translation et/ou le sens de rotation de chaque cycle, le nombre de répétitions et de phases de chaque cycle, la durée de chacune des phases de chacun desdits cycle, et l'intensité de l'accélération de chaque première et/ou troisième phase d'un cycle.

5. Dispositif selon l'une des revendications 3 et 4, caractérisé en ce que l'accélération de la première phase est d'intensité comprise entre 0,1 g et 3 g, et la durée de cette première phase est comprise entre 1 milliseconde et 100 millisecondes,
en ce que la vitesse constante de la seconde phase dépend de la vitesse de l'équipage (4) à la fin de la première phase, et la durée de cette seconde phase est comprise entre 0 seconde et 5 secondes, et
en ce que l'accélération de la troisième phase est d'intensité comprise entre 0,1 g et 3 g, et la durée de cette troisième phase est comprise entre 1 milliseconde et 100 millisecondes.

6. Dispositif selon l'une des revendications 3 à 5, caractérisé en ce que les moyens de traitement (23) sont également aptes à valider un cycle en fonction de critères d'admissibilité sur le mouvement des yeux, la forme et/ou l'amplitude des ondes détectées par les électrodes (18).

7. Dispositif selon la revendication 6, caractérisé en ce que les moyens de traitement (23) sont également aptes à autoriser un n-ième cycle après validation du (n-1)-ième cycle, ainsi qu'à recommencer un n-ième cycle jusqu'à sa validation.

8. Dispositif selon l'une des revendications 6 à 7, caractérisé en ce qu'après validation de chaque cycle, les moyens de traitement (23) sont aptes à délivrer sur un moniteur vidéo (19) au moins une image de type tableau de courbes de potentiels évoqués représentatives de l'amplitude de l'émission des régions du cerveau détectée par chaque électrode (18), en réponse à chaque phase de stimulation du cycle.

9. Dispositif selon la revendication 8, caractérisé en ce que l'image délivrée après un n-ième cycle, d'une succession de cycles identiques, est représentative de la moyenne des amplitudes des n cycles identiques écoulés.

10. Dispositif selon l'une des revendications 3 à 9, caractérisé en ce que les moyens de traitement (23) comprennent également une mémoire de masse (24) apte à stocker dans un fichier l'ensemble des données résultant de chaque cycle d'un examen, lesquelles données forment des résultats d'examen.

11. Dispositif selon la revendication 10, caractérisé en ce que les moyens de traitement (23) sont également aptes à traiter les données contenues dans au moins un fichier d'examen, pour les transformer en données de champs de potentiel ou de densité de courant, et délivrer sur le moniteur vidéo (19) au moins une image , de type représentation tridimensionnelle "3D" de la surface d'une calotte crânienne de sujet, représentative des données résultant d'au moins un n-ième cycle de l'examen.

12. Dispositif selon l'une des revendications 10 et 11, caractérisé en ce que la mémoire de masse (24) est en outre apte à stocker des données relatives à des examens tests formant base de données, pour autoriser au moins une comparaison, sur le moniteur vidéo (19), entre les résultats d'au moins un examen, ou d'au moins un n-ième cycle d'au moins un examen, et les données d'au moins un examen test.

13. Dispositif selon l'une des revendications précédentes, caractérisé en ce que l'équipage (4) comprend en outre un appuie-tête (6) apte à contrôler tout mouvement de tête lors d'un examen, un générateur de bruit blanc apte à contrôler des informations auditives parasites, ainsi qu'un cache visuel apte à contrôler temporairement des informations visuelles, ce qui permet de mieux s'assurer de l'origine vestibulaire de la stimulation,
en ce qu'il comprend au moins deux électrodes aptes à détecter les mouvements horizontaux et verticaux des yeux, et
en ce que les moyens de traitement (23) comprennent également des moyens d'amplification (21) des signaux détectés par les électrodes (18).

## Claims

1. Device for examining a subject, in particular his or her vestibular evoked potentials, of the type comprising:
- a framework (1) accommodating an arrangement (4) which is movable in at least one direction inside the said framework and is able to support and immobilize a subject,
- drive means (9-11) which are able to displace the said arrangement (4) in at least one direction, in order to stimulate the vestibular system of the subject,
- an electrophysiology helmet (17) which can be fitted on the said subject's scalp and is equipped with a plurality of electrodes (18), in chosen respective positions, each electrode being able to detect waves of cerebral activity originating from identifiable regions of the brain,
- mechanical means (20) for controlling the said drive means, as a function of a chosen law of movement,
- and processing means (23) which are able to collect the waves detected by the said electrodes (18) in order to derive evoked potentials therefrom, and to search for those evoked potentials which are in relation with the chosen law of movement,
characterized in that the drive means (9-11) are arranged to permit a displacement in translation along at least one of the three directions defining the space, and/or a displacement in rotation on one of the planes defining the said space, of the said arrangement (4),
in that the mechanical means (20) for controlling the said drive means are arranged to make it possible to apply, to the subject, an elementary stimulation in displacement of monotone space/time characteristics, with a profile which is adjustable in terms of speed and/or acceleration, in at least one of the said three directions of the space in translation and/or in at least one of the said three planes of the space in rotation,
in that the processing means (23) are able to digitize the waves detected by some, at least two, of the said electrodes (18), and are arranged to search these for evoked potentials in relation with the profile of the elementary stimulation,
by which means it is possible to obtain information in real time on the functioning of at least one part of the vestibular system.

2. Device according to Claim 1, characterized in that each evoked potential datum is an amplitude, which can be transformed on command, by the processing means (23), to a potential field or to a current density.

3. Device according to one of Claims 1 and 2, characterized in that the processing means (23) are additionally able to store space/time examination characteristics, each examination being defined by the repetition of at least one cycle of stimulation of at least one elementary stimulation phase chosen from among a first phase in which the arrangement (4) is subjected to an acceleration in a given direction or sense of displacement, a second phase in which the arrangement (4) is subjected to a constant speed of translation or rotation in this same direction or this same sense of displacement, and a third phase in which the arrangement (4) is subjected to a deceleration in the same direction or the same sense of displacement as that of the first and second phases.

4. Device according to Claim 3, characterized in that the space/time characteristics of an examination are the direction of translation and/or the sense of rotation of each cycle, the number of repetitions and phases of each cycle, the duration of each of the phases of each of the said cycles, and the intensity of the acceleration of each first and/or third phase of a cycle.

5. Device according to one of Claims 3 and 4, characterized in that the acceleration of the first phase is of an intensity of between 0.1 g and 3 g, and the duration of this first phase is between 1 millisecond and 100 milliseconds,
in that the constant speed of the second phase depends on the speed of the arrangement (4) at the end of the first phase, and the duration of this second phase is between 0 second and 5 seconds, and
in that the acceleration of the third phase is of an intensity of between 0.1 g and 3 g, and the duration of this third phase is between 1 millisecond and 100 milliseconds.

6. Device according to one of Claims 3 to 5, characterized in that the processing means (23) are also able to validate a cycle as a function of criteria of admissibility regarding the movement of the eyes, the form and/or the amplitude of the waves detected by the electrodes (18).

7. Device according to Claim 6, characterized in that the processing means (23) are also able to authorize an n-th cycle after validation of the (n-1)th cycle, and to restart an n-th cycle until its validation.

8. Device according to one of Claims 6 to 7, characterized in that after validation of each cycle, the processing means (23) are able to deliver on a video monitor (19) at least one image of the type showing a table of curves of evoked potentials representative of the amplitude of the emission of the regions of the brain detected by each electrode (18), in response to each stimulation phase of the cycle.

9. Device according to Claim 8, characterized in that the image delivered after an n-th cycle, of a succession of identical cycles, is representative of the mean of the amplitudes of the n identical cycles which have elapsed.

10. Device according to one of Claims 3 to 9, characterized in that the processing means (23) also comprise a bulk memory (24) able to store, in a file, all the data resulting from each cycle of an examination, which data form examination results.

11. Device according to Claim 10, characterized in that the processing means (23) are also able to process the data contained in at least one examination file, in order to transform them into potential field or current density data, and to deliver on the video monitor (19) at least one image, of the type showing a three-dimensional "3D" representation of the surface of the top of the skull of the subject, representative of the data resulting from at least an n-th cycle of the examination.

12. Device according to one of Claims 10 and 11, characterized in that the bulk memory (24) is additionally able to store data relating to test examinations forming a database, in order to authorize at least one comparison, on the video monitor (19), between the results of at least one examination, or of at least an n-th cycle of at least one examination, and the data from at least one test examination.

13. Device according to one of the preceding claims, characterized in that the arrangement (4) additionally comprises a headrest (6) which is able to control any movement of the head during an examination, a white noise generator which is able to control parasite auditory information, and a viewing mask which is able to temporarily control visual information, by which means it is easier to ensure the vestibular origin of the stimulation,
in that it comprises at least two electrodes which are able to detect the horizontal and vertical movements of the eyes, and
in that the processing means (23) also comprise means (21) for amplifying the signals detected by the electrodes (18).

## Patentansprüche

1. Untersuchungsvorrichtung für eine Person, insbesondere ihrer vestibularen evozierten Potentiale, umfassend:
- einen Rahmen (1) für ein Gerät (4), das im Innern dieses Rahmens in wenigstens einer Richtung beweglich ist und fähig, eine Person zu tragen und unbeweglich zu halten,
- Antriebseinrichtungen (9-11) zum Verschieben des genannten Geräts (4) in wenigstens einer Richtung, um das vestibulare System der Person zu stimulieren,
- einen elektro-physiologischen Helm (17), der sich an den Skalp bzw. Kopf der genannten Person anpaßt und mit mehreren Elektroden (18) in jeweils ausgewählten Positionen ausgestattet ist, wobei jede Elektrode fähig ist, Gehirnaktivitätswellen zu detektieren, die von identifizierbaren Zonen des Gehirns stammen,
- mechanische Steuereinrichtungen (20) der genannten Antriebseinrichtungen, abhängig von einem gewählten Bewegungsgesetz,
sowie Verarbeitungseinrichtungen (23) zum Erfassen der durch die Elektroden 18 detektierten Wellen, um aus ihnen evozierte Potentiale abzuleiten und solche evozierten Potentiale zu untersuchen, die mit dem gewählten Bewegungsgesetz in Verbindung stehen,
**dadurch gekennzeichnet,** daß die Antriebseinheiten (9-11) so angeordnet sind, daß sie eine Translationsbewegung in wenigstens einer der drei den Raum definierenden Richtungen und/oder eine Rotationsbewegung in einer der den genannten Raum definierenden Ebenen des genannten Geräts (4) möglich machen,
dadurch, daß die mechanischen Steuereinrichtungen (20) der genannten Antriebseinrichtungen so angeordnet sind, daß sie die Anwendung einer Bewegungselementarstimulation monotoner räumlichzeitlicher Charakteristika ermöglichen, mit einem justierbaren Profil bezüglich Geschwindigkeit und Beschleunigung in wenigstens einer der drei Raumrichtungen bei Translation und/oder in wenigstens einer der drei Raumrichtungen bei Rotation,
dadurch, daß die Verarbeitungseinrichtungen (23) fähig sind, die durch bestimmte und wenigstens zwei der genannten Elektroden (18) detektierten Wellen zu digitalisieren, angeordnet um entsprechend dem Profil der Elementarstimulation evozierte Potentiale zu untersuchen,
was ermöglicht, in Echtzeit Informationen bezüglich des Arbeitens wenigstens eines Teils des vestibularen Systems zu erhalten.

2. Vorrichtung nach Anspruch 1, dadurch gekennzeichnet, daß jede evozierte Potentialgröße eine Amplitude ist, die durch die Verarbeitungseinrichtungen (23) auf Befehl umwandelbar ist in ein Potenialfeld oder in Stromdichte.

3. Vorrichtung nach einem der Ansprüche 1 und 2, dadurch gekennzeichnet, daß die Verarbeitungseinrichtungen (23) außerdem fähig sind, räumlich-zeitliche Prüfungs- bzw. Untersuchungskenndaten zu speichern, wobei jede Untersuchung definiert wird durch die Wiederholung wenigstens eines Stimulationszyklus von wenigstens einer Elementarstimulationsphase, ausgewählt unter einer ersten Phase, in der das Gerät (4) einer Beschleunigung in einer bestimmte Bewegungsrichtung ausgesetzt ist, einer zweiten Phase, in der das Gerät (4) einer konstanten Translations- oder Rotationsbewegung in dieser selben Bewegungsrichtung ausgesetzt ist, und einer dritte Phase, in der das Gerät (4) einer Verzögerung in derselben Bewegungsrichtung wie derjenigen der ersten und der zweiten Phase ausgesetzt ist.

4. Vorrichtung nach Anspruch 3, dadurch gekennzeichnet, daß die räumlich-zeitlichen Charakteristika einer Prüfung bzw. Untersuchung die Translations- und/oder Rotationsrichtung jedes Zyklus, die Anzahl der Wiederholungen und Phasen jedes Zyklus, die Dauer jeder der Phasen jedes der genannten Zyklen und die Intensität der Beschleunigung der ersten und/oder dritten Phase eines Zyklus sind.

5. Vorrichtung nach einem der Ansprüche 3 und 4,
dadurch gekennzeichnet, daß die Intensität der Beschleunigung der ersten Phase zwischen 0,1 g und 3 g enthalten ist, und die Dauer dieser ersten Phase zwichen 1 ms und 100 ms enthalten ist,
dadurch, daß die konstante Geschwindigkeit der zweiten Phase abhängig ist von der Geschwindigkeit des Geräts (4) am Ende der ersten Phase, und die Dauer dieser zweiten Phase zwischen 0 Sekunden und 5 Sekunden enthalten ist, und
dadurch, daß die Intensität der Beschleunigung bzw. Verzögerung der dritten Phase zwischen 0,1 g und 3 g enthalten ist, und die Dauer dieser dritten Phase zwischen 1 ms und 100 ms enthalten ist.

6. Vorrichtung nach einem der Ansprüche 3 bis 5, dadurch gekennzeichnet, daß die Verarbeitungseinrichtungen (23) auch fähig sind, einen Zyklus in Abhängigkeit von Zulässigkeitskriterien bezüglich der Augenbewegung, der Form und/oder der Amplitude der durch die Elektroden (18) detektierten Wellen zu validieren.

7. Vorrichtung nach Anspruch 6, dadurch gekennzeichnet, daß die Verarbeitungseinrichtungen (23) ebenfalls fähig sind, einen n-ten Zyklus nach Validierung des (n-1)-ten Zyklus zuzulassen sowie einen n-ten Zyklus bis zu seiner Validierung wiederzubeginnen.

8. Vorrichtung nach einem der Ansprüche 6 und 7, dadurch gekennzeichnet, daß nach Validierung jedes Zyklus die Verarbeitungseinrichtungen (23) fähig sind, auf einem Videomonitor (19) wenigstens ein Bild des Typs Kurventafeln von evozierten Potentiale zu liefern, die repräsentativ sind für Amplitude der Emission der durch jede Elektrode (18) detektierten Zonen des Gehirns als Reaktion auf jede Stimulationsphase des Zyklus.

9. Vorrichtung nach Anspruch 8, dadurch gekennzeichnet, daß das nach einem n-ten Zyklus gelieferte Bild einer Folge identischer Zyklen repräsentativ ist für den Durchschnitt der Amplituden der n abgelaufenen identischen Zyklen.

10. Vorrichtung nach einem der Ansprüche 3 bis 9, dadurch gekennzeichnet, daß die Verarbeitungseinrichtungen (23) auch einen Massenspeicher (24) umfassen, der in einer Datei die Gesamtheit der Daten speichern kann, die aus jedem Zyklus einer Untersuchung resultieren, wobei diese Daten Untersuchungsresultate bilden.

11. Vorrichtung nach Anspruch 10, dadurch gekennzeichnet, daß die Verarbeitungseinrichtungen (23) auch fähig sind, die in wenigstens einer Untersuchungsdatei enthaltenen Daten zu verarbeiten, um sie in Potentialfeld- oder Stromdichtedaten umzuwandeln und auf dem Videomonitor (19) wenigstens ein Bild zu liefern, das dreidimensional nach "3D"-Art die Oberfläche einer Schädelkalotte der Person darstellt, das repräsentativ ist für die aus wenigstens einem n-ten Zyklus der Untersuchung stammenden Daten.

12. Vorrichtung nach einem der Ansprüche 10 und 11, dadurch gekennzeichnet, daß der Massenspeicher (24) auch fähig ist, Daten zu speichern, die Testuntersuchungen betreffen und eine Datenbank bilden, um auf dem Videomonitor (19) wenigstens einen Vergleich zwischen den Resultaten von wenigstens einer Untersuchung oder von wenigstens einem n-ten Zyklus wenigstens einer Untersuchung und den Daten von wenigstens einer Testuntersuchung zuzulassen.

13. Vorrichtung nach einem der vorangehenden Ansprüche,
dadurch gekennzeichnet, daß das Gerät (4) außerdem eine Kopfstütze (6) umfaßt, fähig jede Kopfbewegung während einer Untersuchung zu kontrollieren, und einen Rauschstörungsgenerator, fähig Hörstörungsinformationen zu kontrollieren, sowie eine Sehabdeckung, fähig vorübergehend visuelle Informationen zu kontrollieren, was ermöglicht, sich besser des vestibulären Ursprungs der Stimulation zu versichern,
dadurch, daß es wenigstens zwei Elektroden umfaßt, fähig die Horizontal- und Vertikalbewegungen der Augen zu kontrollierten,
und dadurch, daß die Verarbeitungseinrichtungen (23) auch Verstärkungseinrichtungen (21) der durch die Elektroden (18) detektierten Signale umfassen.
